(19) Europäisches Patentamt
European Patent Office
Office européen des brevets

(11) Veröffentlichungsnummer: **0 020 968**
**A1**

(12) # EUROPÄISCHE PATENTANMELDUNG

(21) Anmeldenummer: **80102554.5**

(22) Anmeldetag: **09.05.80**

(51) Int. Cl.³: **C 07 D 249/08**, C 07 D 233/60, C 07 D 231/12, C 07 D 233/68, C 07 D 249/10, C 07 D 231/16, A 01 N 43/50, A 01 N 43/56, A 01 N 43/64, C 07 D 405/12, C 07 D 409/12

(30) Priorität: **09.06.79 DE 2923439**

(43) Veröffentlichungstag der Anmeldung: **07.01.81 Patentblatt 81/1**

(84) Benannte Vertragsstaaten: **AT BE CH DE FR GB IT LI LU NL SE**

(71) Anmelder: **BASF Aktiengesellschaft, Carl-Bosch-Strasse 38, D-6700 Ludwigshafen (DE)**

(72) Erfinder: **Ammermann, Eberhard, Dr., Sachsenstrasse 3, D-6700 Ludwigshafen (DE)**
Erfinder: **Husslein, Gerd, Dr., Rubensstrasse 36, D-6700 Ludwigshafen (DE)**
Erfinder: **Zeeh, Bernd, Dr., Thorwaldsenstrasse 5, D-6700 Ludwigshafen (DE)**
Erfinder: **Hamprecht, Gerhard, Dr., Rote-Turm-Strasse 28, D-6940 Weinheim (DE)**
Erfinder: **Pommer, Ernst-Heinrich, Dr., Berliner Platz 7, D-6703 Limburgerhof (DE)**

(54) **Glykolaldehyd-N,O-acetale, ihre Herstellung und Verwendung als Fungizide.**

(57) Die vorliegende Erfindung betrifft Glykolaldehyd-N,O-acetale der Formel

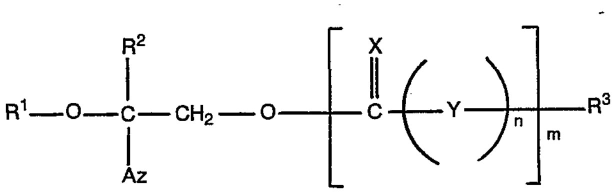

in welcher

- $R^1$ einen gegebenenfalls substituierten Arylrest
- $R^2$ Wasserstoff, einen gegebenenfalls substituierten Alkyl-, Arylalkyl- oder Arylrest
- $R^3$ einen gegebenenfalls substituierten Alkyl-, Alkenyl-, Alkinyl-, Cycloalkyl-, Cycloalkenyl-, Arylalkyl-, Heteroarylalkyl-, Aryl- oder Heteroarylrest bedeutet und für den Fall, daß m = 0 ist, $R^3$ zusätzlich auch Wasserstoff oder einen Trialkylsilylrest bedeutet und
- X für Sauerstoff oder Schwefel
- Y für Sauerstoff, Schwefel oder $NR^4$
- n für 0 oder 1
- m für 0 oder 1
- Az für gegebenenfalls alkyl- oder halogensubstituiertes 1, 2, 4-Triazol, Imidazol oder Pyrazol und
- $R^4$ für Wasserstoff, einen Alkyl- oder Alkoxyrest steht sowie seine Salze und Metallkomplexverbindungen, Verfahren zu ihrer Herstellung und ihre Anwendung als Fungizide.

EP 0 020 968 A1

ACTORUM AG

Glykolaldehyd-N,O-acetale, ihre Herstellung und Verwendung als Fungizide

Die vorliegende Erfindung betrifft neue Glykolaldehyd-N,O-acetale, Verfahren zu ihrer Herstellung, Fungizide, die diese Wirkstoffe enthalten, und Verfahren zur Bekämpfung von Schadpilzen mit diesen Wirkstoffen.

Es ist bekannt, daß das 1-(2'-(2'',4''-Dichlorphenyl-)-2'-(2''-propenyloxy-)-ethyl)-1H-imidazol (GB-PS 13 18 590) eine fungizide Wirkung hat. Seine Wirkung insbesondere gegen Mehltau- und Rostpilze ist jedoch unzureichend. Daher ist es in der Praxis für den Gebrauch als Pflanzenschutzmittel zur Bekämpfung von Schadpilzen wenig geeignet.

Es wurde nun gefunden, daß die neuen Glykolaldehyd-N,O-acetale der allgemeinen Formel

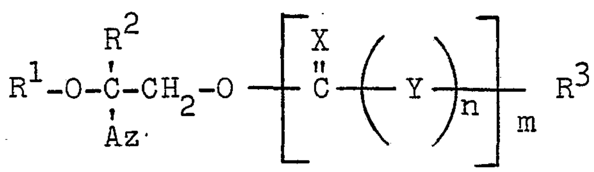
I

in welcher

$R^1$ einen gegebenenfalls substituierten Arylrest

$R^2$ einen Wasserstoff, einen gegebenenfalls substituierten Alkyl-, Arylalkyl- oder Arylrest

$R^3$ einen gegebenenfalls substituierten Alkyl-, Alkenyl-, Alkinyl-, Cycloalkyl-, Cycloalkenyl-, Arylalkyl-, Heteroarylalkyl-, Aryl- oder Heteroarylrest bedeutet und für den Fall, daß m = 0 ist, $R^3$ zusätzlich auch Wasserstoff oder einen Trialkylsilylrest bedeutet und

X für Sauerstoff oder Schwefel

Y für Sauerstoff, Schwefel oder $NR^4$

n für 0 oder 1

Sws/St

m für 0 oder 1

Az für gegebenenfalls alkyl- (z.B. methyl) oder halogensubstituiertes (z.B. durch Chlor) 1,2,4-Triazol, Imidazol oder Pyrazol und

$R^4$ für Wasserstoff, einen Alkyl- (1-4 C-Atome) oder Alkoxyrest (1-4 C-Atome)

steht, und seine Salze und Metallkomplexverbindungen eine ausgezeichnete fungizide Wirksamkeit haben.

In der Formel I steht $R^1$ beispielsweise für einen gegebenenfalls substituierten Arylrest mit 6 bis 12 Kohlenstoffatomen, insbesondere für einen gegebenenfalls substituierten Phenyl- oder Naphthylrest.

Als Substituenten des Arylrestes seien beispielsweise genannt: Halogen, insbesondere Fluor, Chlor oder Brom; Nitro; ein geradkettiger Alkylrest mit 1 bis 10 Kohlenstoffatomen, ein verzweigter oder cyclischer Alkylrest mit 3 bis 10 Kohlenstoffatomen; ein Alkoxy- oder Alkylthiorest mit 1 bis 5 Kohlenstoffatomen, ein Halogenalkyl-, Halogenalkoxy-, Halogenalkylthio- oder Halogenalkylsulfonylrest mit bis zu 4 Kohlenstoffatomen und bis zu 9 Halogenatomen, insbesondere Fluor oder Chlor; ein Alkoxyalkyl- oder Alkylthioalkylrest mit 2 bis 6 Kohlenstoffatomen, beispielsweise ein Ethoxypropyl- oder ein Methylmercaptomethylrest; ein gegebenenfalls substituierter Phenylrest mit 6 bis 11 Kohlenstoffatomen; ein gegebenenfalls insbesondere in der 4-Stellung substituierter Aryloxy-, Arylalkyloxy- oder Arylazorest, beispielsweise ein 4-(4'-Chlorphenoxy)-, 4-(2',4'-Dichlorphenoxy)-, 4-(4'-Trifluormethylphenoxy)-Rest oder ein 4-(4'-Chlorphenylazo)- oder 4-(3'-Fluorphenylazo)-Rest; ein gegebenenfalls substituierter Arylthio-, Arylsulfinyl- oder Arylsulfonylrest; ein gegebenenfalls substituierter Arylalkylrest,

insbesondere ein unsubstituierter oder substituierter Benzylrest; ein geradkettiger Alkenylrest mit 2 bis 6 oder ein verzweigter Alkenylrest mit 3 bis 7 Kohlenstoffatomen; ein geradkettiger Alkinylrest mit 2 bis 4 oder ein verzweigter Alkinylrest mit 3 bis 6 Kohlenstoffatomen; ankondensierte Ringe, insbesondere aus den Klassen der Aromaten und Heterocyclen, beispielsweise ankondensierte gegebenenfalls substituierte Benzo-, Benzofuro- oder Pyridinoreste.

$R^2$ steht beispielsweise für Wasserstoff, für einen geradkettigen Alkylrest mit 1 bis 10 oder für einen verzweigten Alkylrest mit 3 bis 8 Kohlenstoffatomen, für einen gegebenenfalls substituierten Aryl- oder Arylalkylrest mit 6 bis 12 Kohlenstoffatomen im Arylteil und 1 bis 2 Kohlenstoffatomen im Alkylteil.

$R^3$ steht beispielsweise für Wasserstoff, für einen gegebenenfalls substituierten Alkylrest, der beispielsweise unverzweigt sein und aus 1 bis 10 Kohlenstoffatomen bestehen kann wie ein Methyl-, Ethyl-, n-Propyl-, n-Pentyl- oder ein n-Decylrest, oder verzweigt sein und 3 bis 10 Kohlenstoffatomen umfassen kann wie beispielsweise ein Isopropyl-, ein 2-Methyl-propyl-1- oder ein 3,3-Dimethyl-n-butyl-1-rest, $R^3$ steht beispielsweise für einen unverzweigten oder verzweigten Alkenyl- oder Alkinylrest mit 2 bzw. 3 bis 10 Kohlenstoffatomen, wie beispielsweise für einen Propen-2-yl-1-, Propin-2-yl-1- oder für einen Penta-1,3-dien-yl-1-rest oder $R^3$ steht beispielsweise für einen cyclischen Alkyl- oder Alkenylrest mit 3 bis 6 Ringkohlenstoffatomen wie beispielsweise für einen Cyclopropyl-, Cyclobutyl-, Cyclohexen-2-yl-1- oder Cyclohexylrest, $R^3$ steht ferner für einen gegebenenfalls substituierten Arylalkyl-, Heteroarylalkyl-, Aryl- oder Heteroarylrest mit 1 bis 2 Kohlenstoffatomen im Alkylteil und 6 bis 12 Kohlenstoffatomen im Arylteil bzw. 5 bis 6 Ringatomen im Heteroarylteil, von den 1 bis 3 Heteroatome beispielsweise Sauerstoff, Stickstoff oder Schwefel sein können wie beispielsweise für einen gegebenenfalls substituierten Phenyl-, Benzyl-, Phenylethyl-, Naphthyl-, Naphthylmethyl-, Furanyl-, Thienyl-, Thienylmethyl-, Isoxazolyl-, Isoxazolyl-methyl-, Imidazolyl-, Imidazolylmethyl-, Triazolylmethyl-, Pyrazolylmethyl-, Pyridyl- oder für einen Pyrimidinylrest.

Als Substituenten für die für $R^3$ genannten Reste seien beispielsweise genannt: Halogen wie Fluor, Chlor, Brom oder Jod; Nitro- oder Cyanogruppen; Niedrigalkyl; Halogenalkyl-, Alkoxy-, Alkylthio oder Halogenalkyloxyreste mit 1 bis 4 Kohlenstoffatomen und 1 bis 9 Halogenatomen wie beispielsweise Methyl, Trifluormethyl, Methoxy, Methylthio, Ethyl, Ethoxy, Tetrafluorethoxy oder ein n-Propyl oder tert.-Butylrest; Ketogruppen wie beispielsweise in einem Phenacylrest; gegebenenfalls insbesondere in der 4-Stellung substituierte Aryloxy-, Arylalkyloxy- oder Arylthioreste wie beispielsweise ein 4-Chlor-phenoxy, ein 2,4-Dichlorphenoxy oder ein 4-Chlor-phenylthiorest.

Die neuen Glykolaldehyd-N,O-acetale und ihre Derivate besitzen im Acetal-C-Atom ein Asymmetriezentrum und je nach der Beschaffenheit von $R^3$ weitere Asymmetriezentren. Mit üblichen Methoden können die optisch reinen Enantiomeren bzw. Diastereomeren erhalten werden. Sie werden von der Erfindung mit umfaßt. Als Fungizide kann man sowohl die reinen Enantiomeren bzw. die einheitlichen Diastereomeren verwenden wie auch die bei der Synthese üblicherweise anfallenden Gemische.

Die Verbindungen der Formel II

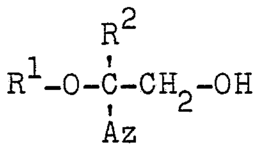

II

in der $R^1$, $R^2$ und Az die oben erläuterten Bedeutungen haben, können hergestellt werden durch Reduktion der Verbindungen der allgemeinen Formel III

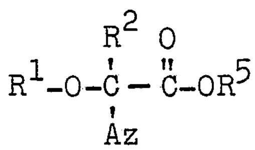

III

worin $R^1$, $R^2$ und Az die oben erläuterten Bedeutungen haben und $R^5$ für Wasserstoff, einen Alkyl- oder Alkoxycarbonylrest mit jeweils 1 bis 4 C-Atome steht, wobei die Reduktion gegebenenfalls in Gegenwart eines Lösungs- oder Verdünnungsmittels, gegebenenfalls in Gegenwart eines Reaktionsbeschleunigers mit komplexen Hydriden oder in Gegenwart eines Katalysators mit Wasserstoff erfolgt.

Die als Ausgangsstoffe benötigten Säurederivate der allgemeinen Formel III sind bekannt.

Zur Synthese der Ester der Formel III kann man zwei verschiedene Herstellungsverfahren benutzen, die in den DE-OS 27 20 654 und 28 46 127 beschrieben werden. Eine direkte Synthese der Säuren wird in der DE-OS 28 46 038 beschrieben.

Die Reduktion der Säuren zu den Alkoholen der Formel II kann nach bekannten Verfahren katalytisch mit Wasserstoff an Adkins-Katalysatoren erfolgen (vergl. B. Cornils und E. Zilly in Methodicum Chimicum Bd. 5, S. 61, Thiemeverlag, Stuttgart, 1975).

Die Säuren, die Ester und die gemischten Anhydride der Formel III können nach den bekannten Methoden der Reduktion mit komplexen Hydriden zum Beispiel $BH_3$, $NaBH_4$ oder $LiAlH_4$ in die Alkohole der Formel II überführt werden (vergl. W.G. Brown in Organic Reactions Vol. VI, S. 469, J. Wiley, New York 1951 und B. Cornils und E. Zilly in Methodicum Chimicum Bd. 5, S. 67).

Ferner wurde gefunden, daß man Verbindungen der Formel I erhält, wenn man eine Verbindung der Formel

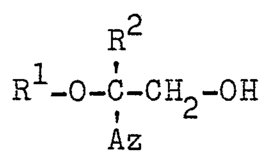 II

oder ihre Alkali-, Erdalkali- oder quartären Ammoniumsalze

a) mit einem Alkylierungsmittel der Formel IV

$R^3L$ IV

oder

b) mit einem Säurederivat der allgemeinen Formel V

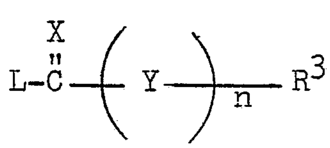 V

oder

c) mit einem Isocyanat der Formel VI umsetzt,

$$R^3-N=C=X \qquad VI$$

worin $R^1$, $R^2$, Az, $R^3$, X, Y und n die oben erläuterten Bedeutungen haben und L für eine nucleophil verdrängbare Abgangsgruppe steht, gegebenenfalls in Gegenwart eines Lösungs- oder Verdünnungsmittels, gegebenenfalls in Gegenwart anorganischer oder organischer Basen, gegebenenfalls unter Zusatz eines Reaktionsbeschleunigers bei Temperaturen zwischen 0 und 120°C umsetzt.

Als nucleophil verdrängbare Abgangsgruppen L seien beispielsweise genannt: Halogen wie Chlor, Brom oder Jod; Alkylsulfat wie Methylsulfat; gegebenenfalls substituierte Alkylsulfonyloxyreste wie Methansulfonyloxy oder Trifluormethansulfonyloxyreste oder Arylsulfonyloxyreste wie der Methylphenylsulfonyloxyrest. Ferner seien als nucleophil verdrängbare Abgangsgruppen L besonders für die Säurederivate der Formel V beispielsweise Azole wie Imidazol oder Triazol und Acyloxygruppen wie in Säureanhydriden genannt.

Geeignete anorganische oder organische Basen, die gegebenenfalls auch als säurebindende Mittel eingesetzt werden können, sind beispielsweise Alkali- und Erdalkalihydroxide wie Natriumhydroxid, Kaliumhydroxid, Calciumhydroxid; Alkalicarbonate wie Kalium- oder Natriumcarbonat; Alkalihydride wie Natriumhydrid; Alkali- oder Erdalkalialkoholate wie Natriummethylat, Magnesiummethylat oder Natriumisopropylat, oder tertiäre Amine wie Trimethylamin, Triethylamin, N,N-Dimethylanilin, N,N-Dimethylcyclohexylamin, N-Methylpiperidin oder Pyridin; Azole wie 1,2,4-Triazol oder Imidazol. Es können aber auch andere übliche Basen verwendet werden.

Mit speziellen Basen wie zum Beispiel Alkalihydriden wie Natriumhydrid oder Lithiumalkylen wie Butyllithium oder mit Alkali- oder Erdalkalialkoholaten wie Natriummethylat können die Alkohole der Formel II auch zunächst in ihre Alkoholat-Salze überführt werden und dann als solche umgesetzt werden.

Zu den Lösungs- bzw. Verdünnungsmitteln gehören beispielsweise Halogenkohlenwasserstoffe wie beispielsweise Methylenchlorid, Chloroform, 1,2-Dichlorethan, Chlorbenzol; aliphatische oder aromatische Kohlenwasserstoffe wie Cyclohexan, Petrolether, Benzol, Toluol oder Xylole; Ester wie Essigsäureethylester; Amide wie Dimethylformamid; Nitrile wie Acetonitril; Sulfoxide, wie Dimethylsulfoxid; Ketone wie Aceton oder Methylethylketon; Ether wie Diethylether, Tetrahydrofuran oder Dioxan oder entsprechende Gemische.

Als Reaktionsbeschleuniger kommen beispielsweise Metallhalogenide wie Kaliumjodid; Kronenether; quartäre Ammoniumverbindungen wie Tetrabutylammoniumjodid oder Säuren oder Kombinationen dieser Reaktionsbeschleuniger in Frage.

Ferner wurde gefunden, daß man Verbindungen der Formel I erhält, wenn man Verbindungen der Formel VII

$$R^1O-\overset{\displaystyle R^2}{\underset{\displaystyle Az}{C}}-CH_2OZ \qquad \text{VII}$$

worin $R^1$, $R^2$ und Az die oben erläuterten Bedeutungen haben und OZ für eine nucleophil verdrängbare Abgangsgruppe steht, mit einer nucleophilen Verbindung der Formel VIII

$$HOR^3 \qquad \text{VIII}$$

worin $R^3$ die oben erläuterte Bedeutung hat, oder mit deren Alkali- oder Erdalkalisalz gegebenenfalls in Gegenwart eines Lösungs- oder Verdünnungsmittels, gegebenenfalls in Gegenwart einer anorganischen oder organischen Base sowie gegebenenfalls in Gegenwart eines Reaktionsbeschleunigers bei Temperaturen zwischen 0 und 120°C umsetzt. Als Beispiele für die erwähnten nucleophil verdrängbaren Abgangsgruppen der Formel OZ seien beispielsweise genannt der Trifluoracetyloxyrest; Alkyloxycarbonyloxyreste, Alkyl- oder Arylsulfonyloxyreste, z.B. die Methansulfonyloxy-, die Trifluormethylsulfonyloxy- oder die Methylphenylsulfonyloxygruppe.

Die Verbindungen der Formel VII können aus den Alkoholen der Formel II durch Umsetzung mit den entsprechenden Säurehalogeniden oder Säureanhydriden nach üblichen Methoden hergestellt werden.

Die Alkali- oder Erdalkalisalze der nucleophilen Verbindung der Formel VIII können auch mit speziellen Basen wie beispielsweise Alkalihydriden, wie Natriumhydrid oder Lithiumalkyl wie Butyllithium oder Alkali- oder Erdalkalialkoholaten wie Natriummethylat zuerst hergestellt und danach umgesetzt werden.

Für die Lösungs- bzw. Verdünnungsmittel, die anorganischen oder organischen Basen sowie für die Reaktionsbeschleuniger gelten die oben gemachten Angaben.

Die oben beschriebenen Umsetzungen werden im allgemeinen bei Temperaturen zwischen 0 und 150°C in einem Zeitraum von 1 bis 60 Stunden durchgeführt, drucklos oder unter Druck, kontinuierlich oder diskontinuierlich.

Zur Abtrennung der neuen Verbindungen von den Reaktionsmischungen wird nach üblichen Methoden vorgegangen. Im allgemeinen bedürfen die so erhaltenen Produkte keiner weiteren Reinigung, sie können aber nach bekannten Methoden wie Umkristallisation, Extraktion, Destillation oder Chromatographie weiter gereinigt werden.

Falls gewünscht werden die Alkohole der Formel II oder die gemäß den oben beschriebenen Verfahren hergestellten Verbindungen der Formel I anschließend nach bekannten Verfahren in ihre für Pflanzen verträglichen Salze oder in ihre Metallkomplexverbindungen überführt.

Als Salze seien insbesondere die nichtphytotoxischen Salze beispielsweise die Säureadditionssalze Hydrochloride, -bromide, -sulfate, -nitrate, -phosphate, -oxalate oder dodecylbenzolsulfonate genannt.

Für die Metallkomplexverbindungen seien die Komplexverbindungen der Alkohole der Formel II oder der Verbindungen der Formel I mit Schwermetallsalzen insbesondere des Zinks, Kupfers, Mangans, Eisens oder Nickels beispielsweise mit Zinkchlorid, Kupfer-II-chlorid, Kupfer-II-sulfat, Mangan-II-chlorid, Eisen-II-chlorid oder Nickel-II-bromid genannt.

Die Herstellung der neuen Verbindungen der Formel I wird durch folgende Beispiele erläutert:

Beispiel 1:

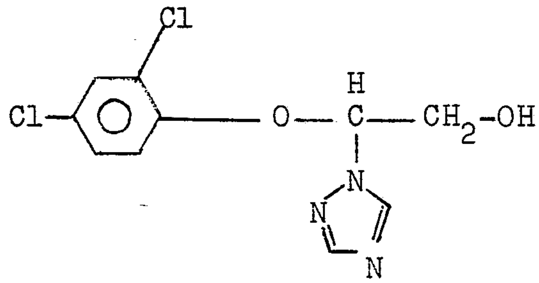

Zu einer Mischung von 115 ml Trimethylborat und 250 ml absolut trockenem Tetrahydrofuran (THF) gibt man 100 g 2-(2',4'-Dichlorphenoxy)-2-(1',2',4'-triazol-1'-yl)-essigsäure und tropft dann bei 25-30°C unter Stickstoff 50 ml Boran-Dimethylsulfid-Komplex zu. Man rührt über Nacht bei Raumtemperatur (20°C) nach, tropft unter Kühlung ca. 100 ml Methanol zu und dampft die Lösung nach Beendigung der Gasentwicklung ein. Der Rückstand wird in Essigester gelöst und mit Wasser und danach mit wenig 1 normaler wäßriger Natronlauge geschüttelt. Nach dem Trocknen und Eindampfen der organischen Phase erhält man 70 g (74 % der Theorie) 2-(2',4'-Dichlorphenoxy)-2-(1',2',4'-triazol-1'-yl)-ethanol (Nr. 1) vom Schmelzpunkt 125-127°C (aus Diisopropylether).

Beispiel 2

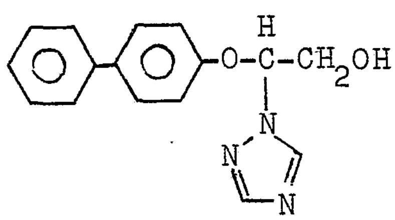

2,8 g Lithiumaluminiumhydrid werden in 50 ml abs. Tetrahydrofuran suspendiert und langsam mit einer Lösung von 35,1 g 2-(4'-Phenylphenoxy-)-2-(1',2',4'-triazol-1'-yl-)-essigsäuretert.-butylester in 150 ml abs. THF versetzt. Nach dem Rühren über Nacht wird das überschüssige $LiAlH_4$ zunächst mit Essigester, dann mit wäßriger Natriumchloridlösung zersetzt. Der entstehende voluminöse Niederschlag wird abgesaugt und gut mit Tetrahydrofuran gewaschen. Die organische Phase wird getrocknet und eingedampft. Der weiße kristalline Rückstand wird zweimal mit Diisopropylether ausgekocht. Man erhält 25 g 2-(4'-Phenylphenoxy-)-2-(1',2',4'-triazol-1'-yl)-ethanol (Nr. 2) vom Schmelzpunkt 95 bis 100°C.

Beispiel 3

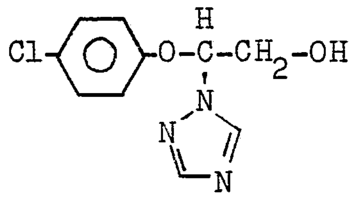

Zu einer Lösung von 21,7 g Chlorkohlensäureethylester in 50 ml abs. Tetrahydrofuran wird bei -5°C eine Lösung des Triethylammoniumsalzes der 2-(4'-Chlorphenoxy)-2-(1',2',4'-triazol-1'-yl)-essigsäure in 300 ml abs. Tetrahydrofuran - hergestellt aus 50,7 g 2-(4'-Chlorphenoxy)-2-(1',2',4'-triazol-1'-yl)-essigsäure und 20,2 g Triethylamin - getropft. Nach halbstündigem Rühren bei -5°C und anschließend bei Raumtemperatur (20°C) wird das ausgefallene Triethylammoniumchlorid abgesaugt und das organische Filtrat bei 10-15°C portionsweise zu einer Lösung von 19 g Natriumborhydrid in 200 ml dest. Wasser gegeben.

Nach dem Rühren über Nacht wird mit verdünnter Salzsäure neutralisiert und das Tetrahydrofuran im Vakuum abgezogen. Die verbleibende wäßrige Phase wird viermal mit je 200 ml $CH_2CH_2$ extrahiert. Die vereinigten organischen Phasen werden nach dem Trocknen eingeengt. Nach Verreiben der zurückbleibenden Kristalle in Diisopropylether erhält man 32 g 2-(4'-Chlorphenoxy-)-2-(1',2',4'-triazol-1'-yl)-ethanol (Nr. 3) vom Schmelzpunkt 108-110°C.

| | C | H | N | Cl |
|---|---|---|---|---|
| ber.: | 50,1 | 4,2 | 17,5 | 14,7 |
| gef.: | 50,3 | 4,5 | 17,2 | 15,0 |

Beispiel 4

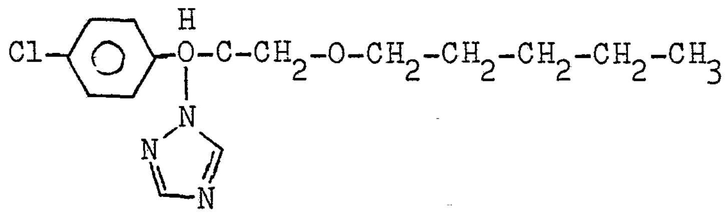

8,9 g 2-(4'-Chlorphenoxy)-2-(1',2',4'-triazol-1'-yl)-ethanol wird mit 32 g n-Pentylchlorid, 32 g einer wäßrigen 50 %igen (Gew.%) Natronlauge und 2 g Tetrabutylammoniumhydrogensulfat versetzt und für 4 Stunden bei 60°C kräftig gerührt. Das Reaktionsgemisch wird mit 500 ml Methylenchlorid ausgeschüttelt, die organische Phase sechsmal mit je 100 ml Wasser gewaschen, getrockent und eingedampft.

Man erhält 6 g des 2-(4'-Chlorphenoxy)-2-(1',2',4'-triazol-1'-yl)-ethyl-1-n-pentyl-ethers (Nr. 4) als farbloses analysenreines Öl.

| | C | H | N | Cl |
|---|---|---|---|---|
| ber.: | 58.1 | 6.5 | 13.5 | 11.4 |
| gef.: | 58.2 | 6.4 | 13.8 | 11.3 |

Beispiel 5

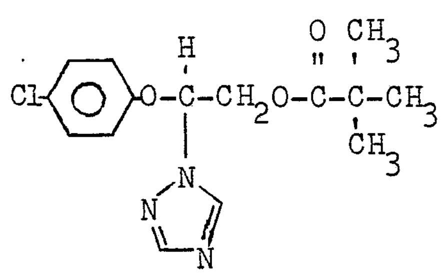

In 150 ml abs. Tetrahydrofuran werden 2,2 g Natriumhydrid suspendiert und mit einer Lösung von 20 g 2-(4'-Chlorphenoxy)-2-(1',2',4'-triazol-1'-yl)-ethanol in 150 ml abs. THF versetzt. Nach einer Stunde Rühren bei Raumtemperatur werden 10 g Pivalinsäurechlord zugetropft. Nach dem Rühren über Nacht werden 20 ml Wasser zugetropft, dann wird das Reaktionsgemisch in 500 ml $CH_2Cl_2$ aufgenommen, mit Wasser gewaschen, getrocknet und eingeengt. Nach Filtration über Kieselgel erhält man 17 g 2,2-Dimethylpropionsäure-[2'-(4''-chlorphenoxy)-2'-(1'',2'',4''-triazol-1''-yl)-ethyl]-ester (Nr. 5) vom Schmelzpunkt 82 bis 84°C.

Beispiel 6

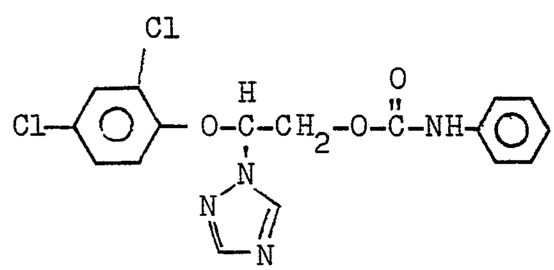

Zu einer Lösung von 22 g 2-(2'-,4'-Dichlorphenoxy)-2-(1',2',4'-triazol-1'-yl) ethanol in 150 ml abs. THF gibt man 9,6 g Phenylisocyanat und rührt 6 Stunden bei Raumtemperatur (20°C) nach. Nach dem Abziehen des Lösungsmittels wird der Rückstand mit Diisopropylether gerührt; man erhält so 25 g (80 % der Theorie) N-Phenyl-[2-(2',4'-dichlorphenoxy)-2-(1',2',4-triazol-1'-yl)-ethyl-1]-carbamat (Nr. 6) vom Schmelzpunkt 105 bis 107°C.

Entsprechend werden beispielsweise folgende Verbindungen der allgemeinen Formel I hergestellt.

Tabelle 1

| Nr. | $R^1$ | $R^2$ | Az | X | Y | n | m | $R^3$ | $n_{20}^D$/Fp |
|---|---|---|---|---|---|---|---|---|---|
| 7 | $4-Cl-C_6H_4-$ | H | 1,2,4-Triazol-1-yl | - | - | 0 | 0 | $CH_3$ | öl |
| 8 | $4-Cl-C_6H_4-$ | H | " | - | - | 0 | 0 | $CH_2-CH=CH_2$ | öl |
| 9 | $4-Cl-C_6H_4-$ | H | " | - | - | 0 | 0 | $CH_2-C\equiv CH$ | 1.5470 |
| 10 | $4-Cl-C_6H_4-$ | H | " | - | - | 0 | 0 | 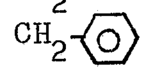 | öl |
| 11 | $4-Cl-C_6H_4-$ | H | " | - | - | 0 | 0 | 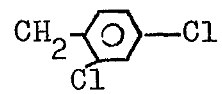 | 55-57 |
| 12 | $4-Cl-C_6H_4-$ | H | " | O | - | 0 | 1 | 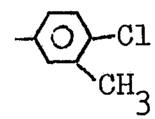 | 87-90 |
| 13 | $2,4-Cl_2-C_6H_3-$ | H | " | - | - | 0 | 0 |  | 68-70 |
| 14 | $2,4-Cl_2-C_6H_3-$ | H | " | - | - | 0 | 0 | $CH_2-CH=CH_2$ | öl |
| 15 | $2,4-Cl_2-C_6H_3-$ | H | " | - | - | 0 | 0 | 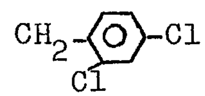 | 61-63 |
| 16 | $2,4-Cl_2-C_6H_3-$ | H | " | O | - | 0 | 1 | 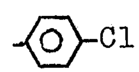 | 87-88 |
| 17 | $2,4-Cl_2-C_6H_3-$ | H | " | O | - | 0 | 1 | $CH_3$<br>$-C-CH_3$<br>$CH_3$ | 92-94 |

| Nr. | $R^1$ | $R^2$ | Az | X | Y | n | m | $R^3$ | $n_{20}^D$/Fp |
|---|---|---|---|---|---|---|---|---|---|
| 18 | $4-Cl-C_6H_4-$ | H | 1,2,4-Triazol-1-yl | – | – | 0 | 0 | $CH_2C(=O)-C(CH_3)(CH_3)-CH_3$ | öl |
| 19 | $4-C_6H_5-C_6H_4-$ | H | " | – | – | 0 | 0 | $CH_3$ | 62-65 |
| 20 | $4-C_6H_5-C_6H_4-$ | H | " | – | – | 0 | 0 | $CH_2C(=O)-C(CH_3)_3$ | öl |
| 21 | $4-C_6H_5-C_6H_4-$ | H | " | – | – | 0 | 0 | 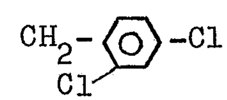 | 84-88 |
| 22 | $4-C_6H_5-C_6H_4-$ | H | " | – | – | 0 | 0 | $CH_2-CH=CH_2$ | |
| 23 | $4-C_6H_5-C_6H_4-$ | H | " | – | – | 0 | 0 | $n-C_5H_{11}$ | 79-80 |
| 24 | $4-C_6H_5-C_6H_4-$ | H | " | O | – | 0 | 1 | $-C(CH_3)_3$ | |
| 25 | $4-C_2-C_6H_4-$ | H | " | O | $NOCH_3$ | 1 | 1 | $CH_3$ | $k_D^{20}$ 1,5171 |
| 26 | $4-C_2-C_6H_4-$ | H | " | O | – | 0 | 1 | 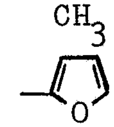 | $n_D^{20}$ 1,5391 |
| 27 | $4-C_2-C_6H_4-$ | H | " | – | – | 0 | 0 | 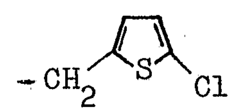 | öl |
| 28 | $4-C_2-C_6H_4-$ | H | " | – | – | 0 | 1 | 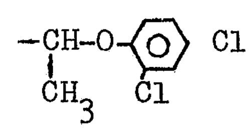 | $n_D^{20}$ 1,5500 |

| Nr. | $R^1$ | $R^2$ | Az | X | Y | n | m | $R^3$ | $n_{20}^{D}$/Fp |
|---|---|---|---|---|---|---|---|---|---|
| 29 | $4-C_2-C_6H_4-$ | H | 1,2,4-Triazol-1-yl | - | - | 0 | 0 | $CH_2-$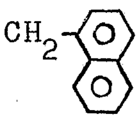 | $n_D^{20}$ 1,5840 |
| 30 | $2,4-Cl_2-C_6H_3-$ | $CH_3$ | " | - | - | 0 | 0 | $CH_3$ | |
| 31 | $2,4-Cl_2-C_6H_3-$ | $C_6H_5$ | " | - | - | 0 | 0 | $CH_2-CH=CH_2$ | |
| 32 | $2,4-Cl_2-C_6H_3-$ | H | Pyrazolyl | - | - | 0 | 0 | H | |
| 33 | $2,4-Cl_2-C_6H_3-$ | H | Imidazolyl | - | - | 0 | 0 | H | |
| 34 | $2,4-Cl_2-C_6H_3-$ | H | " | - | - | 0 | 0 | $CH_3$ | |
| 35 | $2,4-Cl_2-C_6H_3-$ | H | " | O | - | 0 | 1 | $CH_2-OCH_3$ | |
| 36 | $4-Cl-C_6H_4-$ | H | 1,2,4-Triazol-1-yl | - | - | 0 | 0 | $CH_2-$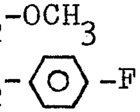$-F$ | 1,5375 |
| 37 | $4-Cl-C_6H_4-$ | H | " | - | - | 0 | 0 | $CH_2-$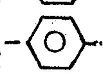$-C(CH_3)_3$ | 1,5240 |
| 38 | $4-C_6H_5-C_6H_4-$ | H | " | - | - | 0 | 0 | $CH_2-$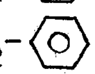$-Cl$ | 1,5950 |
| 39 | $4-C_6H_5-C_6H_4-$ | H | " | - | - | 0 | 0 | $CH_2-$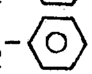$-F$ | 1,5800 |
| 40 | $4-(3F-C_6H_4-N=N)-$ $-C_6H_4)-$ | H | " | - | - | 0 | 0 | H | $145^{o}$(Z) |
| 41 | $C_6H_5-$ | H | " | - | - | 0 | 0 | H | 71-72 |
| 42 | $3-F-C_6H_4$ | H | " | - | - | 0 | 0 | H | 1,5108 |
| 43 | $3-CF_3-C_6H_4-$ | H | " | - | - | 0 | 0 | H | 124-126 |
| 44 | 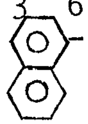- | H | " | - | - | 0 | 0 | H | 116-117 |

| Nr. | $R^1$ | $R^2$ | Az | X | Y | n | m | $R^3$ | $n_{20}^{D}$/Fp |
|---|---|---|---|---|---|---|---|---|---|
| 45 | 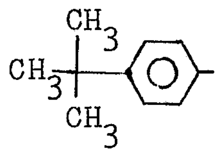 | H | 1,2,4-Triazol-1-yl | - | O | O | | H | 96-98 |
| 46 | 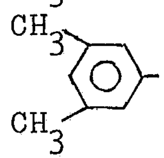 | H | " | - | - | O | O | H | 114-116 |
| 47 | 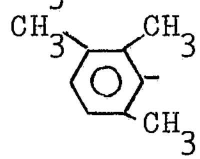 | H | " | - | - | O | O | H | 113-114 |
| 48 | 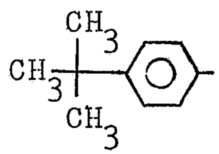 | H | " | - | - | O | O | 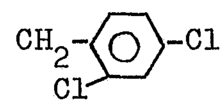 | 1,5562 |
| 49 | $C_6H_5$ | H | " | - | - | O | O | " | 1,5661 |
| 50 | 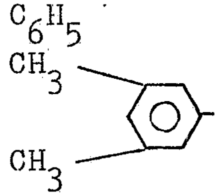 | H | " | - | - | O | O | " | 1,5661 |
| 51 | 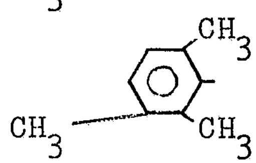 | H | " | - | - | O | O | " | 74-76 |

Die neuen Verbindungen und ihre Salze und Metallkomplexverbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen.

Unter Kulturpflanzen sind in diesem Zusammenhang insbesondere Weizen, Roggen, Gerste, Hafer, Reis, Mais, Baumwolle, Soja, Kaffee, Zuckerrohr, Obst und Zierpflanzen im Gartenbau, sowie Gemüse wie Gurken, Bohnen und Kürbisgewächse zu verstehen.

Die neuen Verbindungen sind insbesondere geeignet zur Bekämpfung folgender Pflanzenkrankheiten:

Erysiphe graminis (echter Mehltau) in Getreide,
Erysiphe cichoriacearum (echter Mehltau) an Kürbisgewächsen,
Podosphaera leucotricha an Äpfeln,
Uncinula necator an Reben,
Erysiphe polygoni an Bohnen,
Sphaerotheca pannosa an Rosen,
Puccinia-Arten an Getreide,
Rhizoctonia solani an Baumwolle sowie
Helminthosphoriumarten an Getreide,
Ustilago-Arten an Getreide und Zuckerrohr,
Rhynchosporium secale an Getreide,
Venturia inaequalis (Apfelschorf),
Mycosphaerella musicola an Bananen.

0020968

Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wir Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen - Fettalkohol - Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95 Gew.% Wirkstoff, vorzugsweise zwischen 0,5 und 90.

Die Aufwandmengen liegen nach Art des gewünschten Effektes zwischen 0,01 und 3, vorzugsweise jedoch zwischen 0,01 und 1 kg Wirkstoff pro Hektar.

Die Mittel bzw. die daraus hergestellten gebrauchsfertigen Zubereitungen, wie Lösugen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung des Beispiels 1 mit 10 Gewichtsteilen N-Methyl- -pyrrolidon und erhält eine Lösung, die zur Anwendung in Form kleinster Tropfen geeignet ist.

II. 20 Gewichtsteile der Verbindung des Beispiels 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100.000 Gewichtsteilen Wasser erhält man eine wäßrige Dispersion, die 0,02 Gew.% des Wirkstoffs enthält.

III. 20 Gewichtsteile der Verbindung des Beispiels 3 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in 100.000 Gewichtsteilen Wasser erhält man eine wäßrige Dispersion, die 0,02 Gew.% des Wirkstoffs enthält.

IV. 20 Gewichtsteile der Verbindung des Beispiels 4 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100.00 Gewichtsteilen Wasser erhält man eine wäßrige Dispersion, die 0,02 Gew.% des Wirkstoffs enthält.

V. 20 Gewichtsteile der Verbindung des Beispiels 1 werden den mit 3 Gewichtsteilen des Natriumsalzes der Diisobutylnaphthalin-α-sulfonsäure, 17 Gewichtsteilen des Natriumsalzes einer Ligninsulfonsäure aus einer Sulfitablauge und 60 Gewichtsteilen pulverförmigem Kieselgel gut vermischt und in einer Hammermühle vermahlen. Durch feines Verteilen der Mischung in 20.000 Gewichtsteilen Wasser erhält man eine Spritzbrühe, die 0,1 Gew.% des Wirkstoffs enthält.

VI. 3 Gewichtsteile der Verbindung des Beispiels 2 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile der Verbindung des Beispiels 3 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese WEise eine Aufbereitung des Wirkstoffs mit guter Haftfähigkeit.

VIII. 40 Gewichtsteile der Verbindung des Beispiels 4 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit 100.000 Gewichtsteilen Wasser erhält man eine wäßrige Dispersion, die 0,04 Gew.% Wirkstoff enthält.

IX. 20 Teile der Verbindung des Beispiels 1 werden mit 2 Teilen Calciumsalz der Dodecylbenzolsulfonsäure, 8 Teilen Fettalkoholpolyglykolether, 2 Teilen Natriumsalz eines Phenolsulfonsäureharnstoff-formaldehyd-Kondensates und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsssprektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Dithiocarbamate und deren Derivate, wie
Ferridimethyldithiocarbamat,
Zinkdimethyldithiocarbamat,
Manganethylenbisdithiocarbamat,
Mangan-Zink-ethylendiamin-bis-dithiocarbamat,

Zinkethylenbisdithiocarbamat,
Tetramethylthiuramdisulfide,
Ammoniak-Komplex von Zink-(N-N-ethylen-bis dithiocarbamat) und
N,N'-Polyäthylen-bis-(thiocarbamoyl)-disulfid,
Zink-(N,N'-propylen-bis-dithiocarbamat),
Ammoniak-Komplex von Zink-(N,N'-propylen-bis-dithiocarbamat) und
N,N'-Polypropylen-bis-(thiocarbamoyl)-disulfid;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;

heterocyclische Strukturen, wie
N-Trichlormethylthio-tetrahydrophthalimid,
N-Trichlormethylthio-phthalimid,
2-Heptadecyl-2-imidazolin-acetat,
2,4-Dichlor-6-(o-chloranilino)-s-triazin,
O,O-Diäthyl-phthalimidophosphonothioat,
5-Amino-1-(bis-(Dimethylamino)-phosphinyl)-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-(2))-benzimidazol,
Piperazin-1,4-diyl-bis-(2,2,2-trichlor-ethyl)-formamid),

2-(Thiazolyl-(4)-benzimidazol,
5-Butyl-2-dimethylamino-4-hydroxy-6-methyl-pyrimidin,
Bis-(p-Chlorphenyl)-3-pyridinmethanol,
1,2-Bis-(3-ethoxycarbonyl-2-thioureido)-benzol,
1,2-Bis-(3-methoxycarbonyl-2-thioureido)-benzol,

und verschiedene Fungizide, wie
Dodecylguanidinacetat,
3-(3-(3,5-Dimethyl-2-oxycyclohexyl)-2-hydroxyäthyl)-glutaramid,
Hexachlorbenzol,
N-Dichlorfluormethylthio-N',N'-dimethyl-N-phenyl-schwefelsäurediamid,
2,5-Dimethyl-furan-3-carbonsäureanilid,
2,5-Dimetyhl-furan-3-carbonsäure-cyclohexylamid,
2-Methyl-benzoesäure-anilid,
2-Jod-benzoesäure-anilid,
1-(3,4-Dichloranilino)-1-formylamino-2,2,2-trichloräthan,
2,6-Dimethyl-N-tridecyl-morpholin bzw. dessen Salze,
2,6-Dimethyl-N-cyclododecyl-morpholin bzw. dessen Salze.

DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
5-Nitro-isophthalsäure-di-isopropylester,
1-(1',2',4'-Triazolyl-1')- 1-(4'-chlorphenoxy) -3,3-dimethyl-butan-2-on,
1-(1',2',4'-Triazolyl-1')- 1-(4'-chlorphenoxy) -3,3-dimethylbutan-2-ol,
N-(2,6-Dimethylphenyl)-N-chloracetyl-D,L-2-aminobutyrolacton,
N-(n-Propyl)-N-(2,4,6-trichlorphenoxyethyl)-N'-imidazolylharnstoff.

Schwefel
N-(1,1,2,2-Tetrachloräthylthio)-tetrahydrophthalimid
2-Cyan-N-(äthylaminocarbonyl)-2-(methoxyimino)-acetamid.
Für den folgenden Versuch wurde das aus der GB-PS 1 318 590 bekannte 1-(2'-(2",4"-Dichlorphenyl)-2'-(2"-propenyloxy)-ethyl-1H-imidazol (Wirkstoff A) verwendet.

Versuch

Wirkung gegen Weizenmehltau

Blätter von in Töpfen gewachsenen Weizenkeimlingen der Sorte "Jubilar" werden mit wäßrigen Emulsionen aus 80 % (Gewichtsprozent) Wirkstoff und 20 % Emulgiermittel besprüht und nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80 % relativer Luftfeuchtigkeit aufgestellt. Nach 10 Tagen wird das Ausmaß der Mehltauentwicklung ermittelt.

| Wirkstoff | Befall der Blätter nach Spritzung mit .. %iger Wirkstoffbrühe | | |
|---|---|---|---|
| | 0,025 | 0,012 | 0,006 |
| 4 | 0 | 0 | 0 |
| 5 | 0 | 0 | 2 |
| 3 | 0 | 1 | 1 |
| 9 | 0 | 0 | 2 |
| 11 | 0 | 0 | 0 |
| 13 | 0 | 0 | 2 |
| 15 | 0 | 0 | 0 |
| 19 | 0 | 0 | 0 |
| 21 | 0 | 0 | 0 |
| 36 | 0 | 0 | 1 |
| 38 | 0 | 0 | 0 |
| 39 | 0 | 0 | 0 |
| 46 | 0 | 1 | 2-3 |
| A | 2 | 3 | 4 |

0 = kein Pilzbefall, abgestuft bis 5 = Totalbefall

Patentansprüche

1. Glykolaldehyd-N,O-acetal der allgemeinen Formel

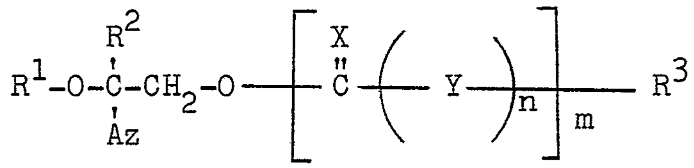

in welcher

$R^1$ einen gegebenenfalls substituierten Arylrest

$R^2$ Wasserstoff, einen gegebenenfalls substituierten Alkyl-, Arylalkyl- oder Arylrest

$R^3$ einen gegebenenfalls substituierten Alkyl-, Alkenyl-, Alkinyl-, Cycloalkyl-, Cycloalkenyl-, Arylalkyl-, Heteroarylalkyl-, Aryl- oder Heteroarylrest bedeutet und für den Fall, daß m = 0 ist, $R^3$ zusätzlich auch Wasserstoff oder einen Trialkylsilylrest bedeutet und

X für Sauerstoff oder Schwefel

Y für Sauerstoff, Schwefel oder $NR^4$

n für 0 oder 1

m für 0 oder 1

Az für gegebenenfalls alkyl- oder halogensubstituiertes 1,2,4-Triazol, Imidazol oder Pyrazol und

$R^4$ für Wasserstoff, einen Alkyl- oder Alkoxyrest steht sowie seine Salze und Metallkomplexverbindungen.

2. Fungizid, enthaltend ein Glykolaldehyd-N,O-acetal der allgemeinen Formel

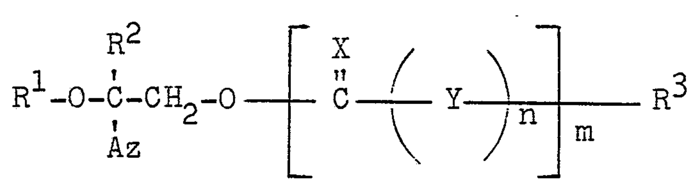

in welcher

$R^1$ einen gegebenenfalls substituierten Arylrest

$R^2$ Wasserstoff, einen gegebenenfalls substituierten Alkyl-, Arylalkyl- oder Arylrest

$R^3$ einen gegebenenfalls substituierten Alkyl-, Alkenyl-, Alkinyl-, Cycloalkyl-, Cycloalkenyl-, Arylalkyl-, Heteroarylalkyl-, Aryl- oder Heteroarylrest bedeutet und für den Fall, daß m = 0 ist, $R^3$ zusätzlich auch Wasserstoff oder einen Trialkylsilylrest bedeutet und

X für Sauerstoff oder Schwefel

Y für Sauerstoff, Schwefel oder $NR^4$

n für 0 oder 1

m für 0 oder 1

Az für gegebenenfalls alkyl- oder halogensubstituiertes 1,2,4-Triazol, Imidazol oder Pyrazol und

$R^4$ für Wasserstoff, einen Alkyl- oder Alkoxyrest steht sowie seine Salze und Metallkomplexverbindungen.

3. Fungizid, enthaltend einen festen oder flüssigen Trägerstoff und ein Glykolaldehyd-N,O-acetal der allgemeinen Formel

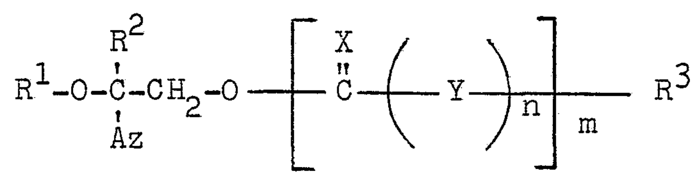

in welcher

$R^1$ einen gegebenenfalls substituierten Arylrest

$R^2$ Wasserstoff, einen gegebenenfalls substituierten Alkyl-, Arylalkyl- oder Arylrest

$R^3$ einen gegebenenfalls substituierten Alkyl-, Alkenyl-, Alkinyl-, Cycloalkyl-, Cycloalkenyl-, Arylalkyl-, Heteroarylalkyl-, Aryl- oder Heteroarylrest bedeutet und für den Fall, daß m = 0 ist,

$R^3$ zusätzlich auch Wasserstoff oder einen Trialkylsilylrest bedeutet und

X für Sauerstoff oder Schwefel

Y für Sauerstoff, Schwefel oder $NR^4$

n für 0 oder 1

m für 0 oder 1

Az für gegebenenfalls alkyl- oder halogensubstituiertes 1,2,4-Triazol, Imidazol oder Pyrazol und

$R^4$ für Wasserstoff, einen Alkyl- oder Alkoxyrest

steht sowie seine Salze und Metallkomplexverbindungen.

4. Verfahren zur Herstellung eines Fungizids, dadurch gekennzeichnet, daß man einen festen oder flüssigen Trägerstoff vermischt mit einem Glykolaldehyd-N,O-acetal der allgemeinen Formel

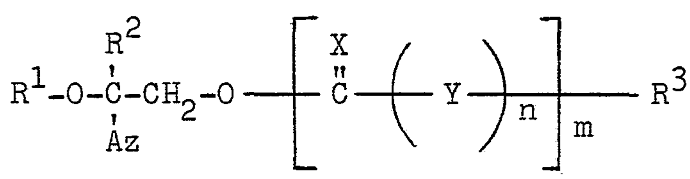

in welcher

$R^1$ einen gegebenenfalls substituierten Arylrest

$R^2$ Wasserstoff, einen gegebenenfalls substituierten Alkyl-, Arylalkyl- oder Arylrest

$R^3$ einen gegebenenfalls substituierten Alkyl-, Alkenyl-, Alkinyl-, Cycloalkyl-, Cycloalkenyl-, Arylalkyl-, Heteroarylalkyl-, Aryl- oder Heteroarylrest bedeutet und für den Fall, daß m = 0 ist, $R^3$ zusätzlich auch Wasserstoff oder einen Trialkylsilylrest bedeutet und

X für Sauerstoff oder Schwefel

Y für Sauerstoff, Schwefel oder $NR^4$

n für 0 oder 1

m für 0 oder 1

Az für gegebenenfalls alkyl- oder halogensubstituiertes 1,2,4-Triazol, Imidazol oder Pyrazol und

$R^4$ für Wasserstoff, einen Alkyl- oder Alkoxyrest steht sowie seine Salze und Metallkomplexverbindungen.

5. Verfahren zur Bekämpfung von Pilzen, dadurch gekennzeichnet, daß man die Pilze oder die vor Pilzbefall zu schützenden Gegenstände behandelt mit einem Glykolaldehyd-N,O-acetal der allgemeinen Formel

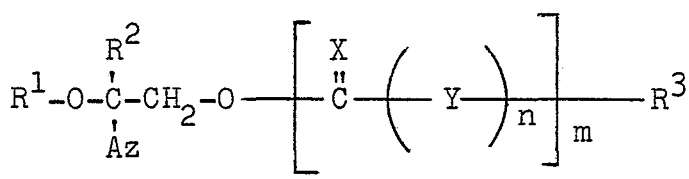

in welcher

$R^1$ einen gegebenenfalls substituierten Arylrest

$R^2$ Wasserstoff, einen gegebenenfalls substituierten Alkyl-, Arylalkyl- oder Arylrest

$R^3$ einen gegebenenfalls substituierten Alkyl-, Alkenyl-, Alkinyl-, Cycloalkyl-, Cycloalkenyl-, Arylalkyl-, Heteroarylalkyl-, Aryl- oder Heteroarylrest bedeutet und für den Fall, daß m = 0 ist, $R^3$ zusätzlich auch Wasserstoff oder einen Trialkylsilylrest bedeutet und

X für Sauerstoff oder Schwefel

Y für Sauerstoff, Schwefel oder $NR^4$

n für 0 oder 1

m für 0 oder 1

Az für gegebenenfalls alkyl- oder halogensubstituiertes 1,2,4-Triazol, Imidazol oder Pyrazol und

$R^4$ für Wasserstoff, einen Alkyl- oder Alkoxyrest steht sowie seine Salze und Metallkomplexverbindungen.

6. Verfahren zur Herstellung eines Glykolaldehyd-N,O-acetal der allgemeinen Formel

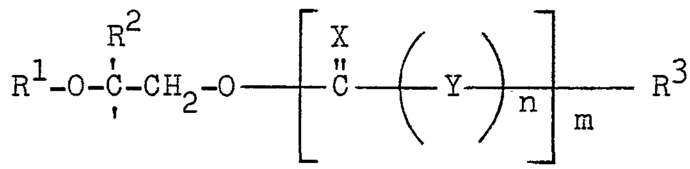

in welcher

$R^1$ einen gegebenenfalls substituierten Arylrest

$R^2$ Wasserstoff, einen gegebenenfalls substituierten Alkyl-, Arylalkyl- oder Arylrest

$R^3$ einen gegebenenfalls substituierten Alkyl-, Alkenyl-, Alkinyl-, Cycloalkyl-, Cycloalkenyl-, Arylalkyl-, Heteroarylalkyl-, Aryl- oder Heteroarylrest bedeutet und für den Fall, daß m = 0 ist, $R^3$ zusätzlich auch Wasserstoff oder einen Trialkylsilylrest bedeutet und

X für Sauerstoff oder Schwefel

Y für Sauerstoff, Schwefel oder $NR^4$

n für 0 oder 1

m für 0 oder 1

Az für gegebenenfalls alkyl- oder halogensubstituiertes 1,2,4-Triazol, Imidazol oder Pyrazol und

$R^4$ für Wasserstoff, einen Alkyl- oder Alkoxyrest steht sowie seine Salze und Metallkomplexverbindungen,

dadurch gekennzeichnet, daß man eine Verbindung der Formel

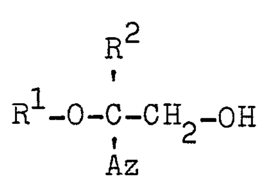

in welcher $R^1$, $R^2$ und Az die oben genannten Bedeutungen haben, oder ihre Alkali- oder Erdalkali- oder quartären Ammoniumsalze

a) mit einem Alkylierungsmittel der allgemeinen Formel

$$R^3 - L$$

oder

b) mit einem Säurederivat der allgemeinen Formel

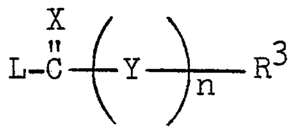

oder

c) mit einem Isocyanat der allgemeinen Formel

$$X=C=N-R^3$$

worin $R^3$, X, Y und n die oben genannten Bedeutungen haben und L für eine nucleophil verdrängbare Abgangsgruppe steht, gegebenenfalls in Gegenwart eines Lösungs- oder Verdünnungsmittels, gegebenenfalls in Gegenwart anorganischer oder organischer Basen, gegebenenfalls in Gegenwart eines Reaktionsbeschleunigers bei Temperaturen zwischen 0 und 120°C umsetzt.

7. Verfahren zur Herstellung eines Glykolaldehyd-N,O--acetal der allgemeinen Formel

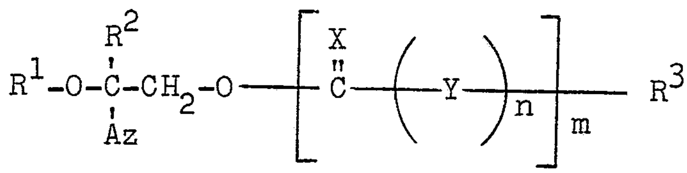

in welcher

$R^1$ einen gegebenenfalls substituierten Arylrest

$R^2$ Wasserstoff, einen gegebenenfalls substituierten Alkyl-, Arylalkyl- oder Arylrest

$R^3$ einen gegebenenfalls substituierten Alkyl-, Alkenyl-, Alkinyl-, Cycloalkyl-, Cycloalkenyl-, Arylalkyl-, Heteroarylalkyl-, Aryl- oder Heteroarylrest bedeutet und für den Fall, daß m = 0 ist, $R^3$ zusätzlich auch Wasserstoff oder einen Trialkylsilylrest bedeutet und

X für Sauerstoff oder Schwefel

Y für Sauerstoff, Schwefel oder $NR^4$

n für 0 oder 1

m für 0 oder 1

Az für gegebenenfalls alkyl- oder halogensubstituiertes 1,2,4-Triazol, Imidazol oder Pyrazol und

$R^4$ für Wasserstoff, einen Alkyl- oder Alkoxyrest steht sowie seine Salze und Metallkomplexverbindungen,

dadurch gekennzeichnet, daß man eine Verbindung der Formel

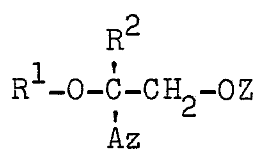

worin $R^1$, $R^2$ und Az die im Anspruch 1 angegebenen Bedeutungen haben und OZ für eine nucleophil verdrängbare Abgangsgruppe steht, mit einer Verbindung der allgemeinen Formel

$$HOR^3$$

worin $R^3$ die im Anspruch 1 genannten Bedeutungen hat, oder mit deren Alkali- oder Erdalkalisalz, gegebenenfalls in Gegenwart eines Lösungs- oder Verdünnungsmittels gegebenenfalls in Gegenwart einer anorganischen oder organischen Base, gegebenenfalls in Gegenwart eines Reaktionsbeschleunigers bei Temperaturen zwischen 0 und 120°C umsetzt.

8. Glykolaldehyd-N,O-acetal ausgewählt aus der Gruppe bestehend aus 2-(4'-Chlorphenoxy)-2-(1',2',4'-triazol-1'-yl-)ethyl-1-n-pentylether, 2-(4'-Chlorphenoxy)-2-(1',2',4'-triazol-1'-yl-)ethyl-1-(2',4'-dichlorbenzyl-)ether, 2,2-Dimethylpropionsäure-[2'-(4''-Chlorphenoxy-)-2'(1'',2'',4''-triazol-1''-yl-)ethyl-1'-]-ester, 2-(2',4'-Dichlorphenoxy-)-2-(1',2',4'-triazol-1'-yl-)-ethyl-1-methylether und 2-(2',4'-Dichlorphenoxy-)-2-(1',2',4'-triazol-1'-yl-)ethyl-1-(2',4'-dichlorbenzyl-)ether.

9. Fungizid, enthaltend ein Glykolaldehyd-N,O-acetal ausgewählt aus der Gruppe bestehend aus 2-(4'-Chlorphenoxy)-2-(1',2',4'-triazol-1'-yl-)ethyl-1-n-pentylether, 2-(4'-Chlorphenoxy)-2-(1',2',4'-triazol-1'-yl-)ethyl-1-(2',4'-dichlorbenzyl-)ether, 2,2-Dimethylpropionsäure-[2'-(4''-Chlorphenoxy-)-2'(1'',2'',4''-triazol-1''-yl-)ethyl-1'-]-ester, 2-(2',4'-Dichlorphenoxy-)-2-(1',2',4'-triazol-1'-yl-)-ethyl-1-methylether und 2-(2',4'-Dichlorphenoxy-)-2-(1',2',4'-triazol-1'-yl-)ethyl-1-(2',4'-dichlorbenzyl-)ether.

## Patentansprüche

1. Fungizid, enthaltend ein Glykolaldehyd-N,O-acetal der allgemeinen Formel

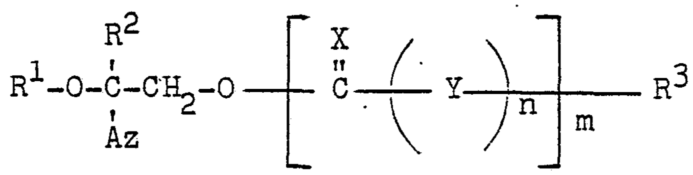

in welcher
$R^1$ einen gegebenenfalls substituierten Arylrest
$R^2$ Wasserstoff, einen gegebenenfalls substituierten Alkyl-, Arylalkyl- oder Arylrest
$R^3$ einen gegebenenfalls substituierten Alkyl-, Alkenyl-, Alkinyl-, Cycloalkyl-, Cycloalkenyl-, Arylalkyl-, Heteroarylalkyl-, Aryl- oder Heteroarylrest bedeutet und für den Fall, daß m = 0 ist, $R^3$ zusätzlich auch Wasserstoff oder einen Trialkylsilylrest bedeutet und
X für Sauerstoff oder Schwefel
Y für Sauerstoff, Schwefel oder $NR^4$
n für 0 oder 1
m für 0 oder 1
Az für gegebenenfalls alkyl- oder halogensubstituiertes 1,2,4-Triazol, Imidazol oder Pyrazol und
$R^4$ für Wasserstoff, einen Alkyl- oder Alkoxyrest steht sowie seine Salze und Metallkomplexverbindungen.

2. Fungizid, enthaltend einen festen oder flüssigen Trägerstoff und ein Glykolaldehyd-N,O-acetal der allgemeinen Formel

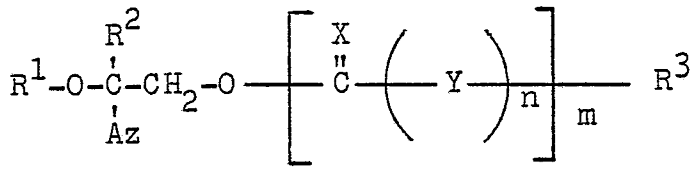

in welcher

$R^1$ einen gegebenenfalls substituierten Arylrest

$R^2$ Wasserstoff, einen gegebenenfalls substituierten Alkyl-, Arylalkyl- oder Arylrest

$R^3$ einen gegebenenfalls substituierten Alkyl-, Alkenyl-, Alkinyl-, Cycloalkyl-, Cycloalkenyl-, Arylalkyl-, Heteroarylalkyl-, Aryl- oder Heteroarylrest bedeutet und für den Fall, daß m = 0 ist, $R^3$ zusätzlich auch Wasserstoff oder einen Trialkylsilylrest bedeutet und

X für Sauerstoff oder Schwefel

Y für Sauerstoff, Schwefel oder $NR^4$

n für 0 oder 1

m für 0 oder 1

Az für gegebenenfalls alkyl- oder halogensubstituiertes 1,2,4-Triazol, Imidazol oder Pyrazol und

$R^4$ für Wasserstoff, einen Alkyl- oder Alkoxyrest steht sowie seine Salze und Metallkomplexverbindungen.

3. Verfahren zur Herstellung eines Fungizids, dadurch gekennzeichnet, daß man einen festen oder flüssigen Trägerstoff vermischt mit einem Glykolaldehyd-N,O-acetal der allgemeinen Formel

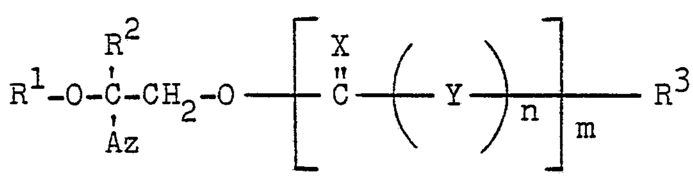

in welcher

$R^1$ einen gegebenenfalls substituierten Arylrest

$R^2$ Wasserstoff, einen gegebenenfalls substituierten Alkyl-, Arylalkyl- oder Arylrest

$R^3$ einen gegebenenfalls substituierten Alkyl-, Alkenyl-, Alkinyl-, Cycloalkyl-, Cycloalkenyl-, Arylalkyl-, Heteroarylalkyl-, Aryl- oder Heteroarylrest bedeutet und für den Fall, daß m = 0 ist, $R^3$ zusätzlich auch Wasserstoff oder einen Trialkylsilylrest bedeutet und

X für Sauerstoff oder Schwefel

Y für Sauerstoff, Schwefel oder $NR^4$

n für 0 oder 1

m für 0 oder 1

Az für gegebenenfalls alkyl- oder halogensubstituiertes 1,2,4-Triazol, Imidazol oder Pyrazol und

$R^4$ für Wasserstoff, einen Alkyl- oder Alkoxyrest steht sowie seine Salze und Metallkomplexverbindungen.

4. Verfahren zur Bekämpfung von Pilzen, <u>dadurch gekennzeichnet</u>, daß man die Pilze oder die vor Pilzbefall zu schützenden Gegenstände behandelt mit einem Glykolaldehyd-N,O-acetal der allgemeinen Formel

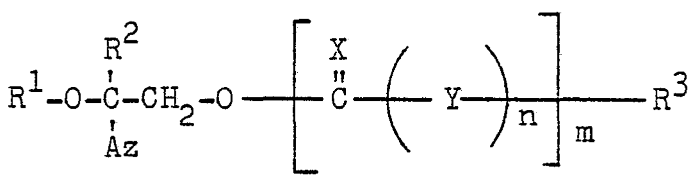

in welcher

$R^1$ einen gegebenenfalls substituierten Arylrest

$R^2$ Wasserstoff, einen gegebenenfalls substituierten Alkyl-, Arylalkyl- oder Arylrest

$R^3$ einen gegebenenfalls substituierten Alkyl-, Alkenyl-, Alkinyl-, Cycloalkyl-, Cycloalkenyl-, Arylalkyl-, Heteroarylalkyl-, Aryl- oder Heteroarylrest bedeutet und für den Fall, daß m = 0 ist, $R^3$ zusätzlich auch Wasserstoff oder einen Trialkylsilylrest bedeutet und

X für Sauerstoff oder Schwefel

Y für Sauerstoff, Schwefel oder $NR^4$

n für 0 oder 1

m für 0 oder 1

Az für gegebenenfalls alkyl- oder halogensubstituiertes 1,2,4-Triazol, Imidazol oder Pyrazol und

$R^4$ für Wasserstoff, einen Alkyl- oder Alkoxyrest steht sowie seine Salze und Metallkomplexverbindungen.

5. Verfahren zur Herstellung eines Glykolaldehyd-N,O-acetal der allgemeinen Formel

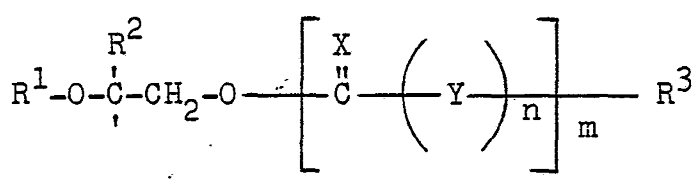

in welcher

$R^1$ einen gegebenenfalls substituierten Arylrest

$R^2$ Wasserstoff, einen gegebenenfalls substituierten Alkyl-, Arylalkyl- oder Arylrest

$R^3$ einen gegebenenfalls substituierten Alkyl-, Alkenyl-, Alkinyl-, Cycloalkyl-, Cycloalkenyl-, Arylalkyl-, Heteroarylalkyl-, Aryl- oder Heteroarylrest bedeutet und für den Fall, daß m = 0 ist, $R^3$ zusätzlich auch Wasserstoff oder einen Trialkylsilylrest bedeutet und

X für Sauerstoff oder Schwefel

Y für Sauerstoff, Schwefel oder $NR^4$

0020968

n für 0 oder 1

m für 0 oder 1

Az für gegebenenfalls alkyl- oder halogensubstituiertes 1,2,4-Triazol, Imidazol oder Pyrazol und

$R^4$ für Wasserstoff, einen Alkyl- oder Alkoxyrest steht sowie seine Salze und Metallkomplexverbindungen,

dadurch gekennzeichnet, daß man eine Verbindung der Formel

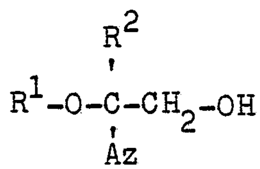

in welcher $R^1$, $R^2$ und Az die oben genannten Bedeutungen haben, oder ihre Alkali- oder Erdalkali- oder quartären Ammoniumsalze

a) mit einem Alkylierungsmittel der allgemeinen Formel

$$R^3 - L$$

oder

b) mit einem Säurederivat der allgemeinen Formel

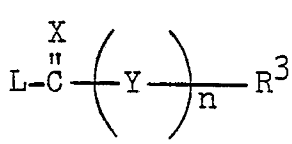

oder

c) mit einem Isocyanat der allgemeinen Formel

$$X=C=N-R^3$$

worin $R^3$, X, Y und n die oben genannten Bedeutungen haben und L für eine nucleophil verdrängbare Abgangsgruppe steht, gegebenenfalls in Gegenwart eines Lösungs- oder Verdünnungsmittels, gegebenenfalls in Gegenwart anorganischer oder organischer Basen, gegebenenfalls in Gegenwart eines Reaktionsbeschleunigers bei Temperaturen zwischen 0 und 120°C umsetzt.

6. Verfahren zur Herstellung eines Glykolaldehyd-N,O--acetal der allgemeinen Formel

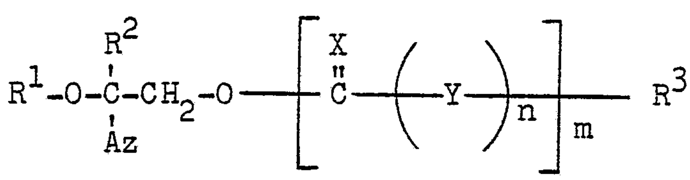

in welcher

$R^1$ einen gegebenenfalls substituierten Arylrest

$R^2$ Wasserstoff, einen gegebenenfalls substituierten Alkyl-, Arylalkyl- oder Arylrest

$R^3$ einen gegebenenfalls substituierten Alkyl-, Alkenyl-, Alkinyl-, Cycloalkyl-, Cycloalkenyl-, Arylalkyl-, Heteroarylalkyl-, Aryl- oder Heteroarylrest bedeutet und für den Fall, daß m = 0 ist, $R^3$ zusätzlich auch Wasserstoff oder einen Trialkylsilylrest bedeutet und

X für Sauerstoff oder Schwefel

Y für Sauerstoff, Schwefel oder $NR^4$

n für 0 oder 1

m für 0 oder 1

Az für gegebenenfalls alkyl- oder halogensubstituiertes 1,2,4-Triazol, Imidazol oder Pyrazol und

$R^4$ für Wasserstoff, einen Alkyl- oder Alkoxyrest steht sowie seine Salze und Metallkomplexverbindungen,

<u>dadurch gekennzeichnet</u>, daß man eine Verbindung der Formel

$$R^1-O-\overset{\overset{\displaystyle R^2}{|}}{\underset{\underset{\displaystyle Az}{|}}{C}}-CH_2-OZ$$

worin $R^1$, $R^2$ und Az die im Anspruch 1 angegebenen Bedeutungen haben und OZ für eine nucleophil verdrängbare Abgangsgruppe steht, mit einer Verbindung der allgemeinen Formel

$$HOR^3$$

worin $R^3$ die im Anspruch 1 genannten Bedeutungen hat, oder mit deren Alkali- oder Erdalkalisalz, gegebenenfalls in Gegenwart eines Lösungs- oder Verdünnungsmittels gegebenenfalls in Gegenwart einer anorganischen oder organischen Base, gegebenenfalls in Gegenwart eines Reaktionsbeschleunigers bei Temperaturen zwischen 0 und 120oC umsetzt.

7. Fungizid, enthaltend ein Glykolaldehyd-N,O-acetal ausgewählt aus der Gruppe bestehend aus 2-(4'-Chlorphenoxy)-2-(1',2',4'-triazol-1'-yl-)ethyl-1-n-pentylether, 2-(4'-Chlorphenoxy)-2-(1',2',4'-triazol-1'-yl-)ethyl-1-(2',4'-dichlorbenzyl-)ether, 2,2-Dimethylpropionsäure-[2'-(4''-Chlorphenoxy-)-2'(1'',2'',4''-triazol-1''-yl-)ethyl-1'-]-ester, 2-(2',4'-Dichlorphenoxy-)-2-(1',2',4'-triazol-1'-yl-)-ethyl-1-methylether und 2-(2',4'-Dichlorphenoxy-)-2-(1',2',4'-triazol-1'-yl-)ethyl-1-(2',4'-dichlorbenzyl-)ether.

Europäisches Patentamt

# EUROPÄISCHER RECHERCHENBERICHT

0020968

Nummer der Anmeldung

EP 80 10 2554

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | DE - A - 27 20 949 (BAYER) | 1,2,3, 4,5,6 |
| A | DE - A - 26 04 761 (BAYER) | 1,2,3, 4,5,6 |
| A | DE - A - 26 04 865 (BAYER) | 1,2,3, 4,5,6 |
| | ------ | |

KLASSIFIKATION DER ANMELDUNG (Int. Cl.³)

C 07 D 249/08
233/60
231/12
233/68
249/10
231/16
A 01 N 43/50
43/56
43/64
C 07 D 405/12
409/12

RECHERCHIERTE SACHGEBIETE (Int. Cl.³)

C 07 D 249/08
233/60

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 08-09-1980 | CREMERS |